(19) **Europäisches Patentamt / European Patent Office / Office européen des brevets**

(11) **EP 1 082 098 B1**

(12) **FASCICULE DE BREVET EUROPEEN**

(45) Date de publication et mention
de la délivrance du brevet:
**06.03.2019 Bulletin 2019/10**

(51) Int Cl.:
*A61K 8/365* (2006.01)   *A61K 8/44* (2006.01)
*A61Q 19/08* (2006.01)   *A61K 8/97* (2017.01)

(21) Numéro de dépôt: **99922237.5**

(22) Date de dépôt: **28.05.1999**

(86) Numéro de dépôt international:
**PCT/FR1999/001261**

(87) Numéro de publication internationale:
**WO 1999/062481 (09.12.1999 Gazette 1999/49)**

(54) **PROCEDE DE TRAITEMENT COSMETIQUE POUR LUTTER CONTRE LES EFFETS DU VIEILLISSEMENT CUTANE**

KOSMETISCHES BEHANDLUNGSVERFAHREN FÜR DIE BEKÄMPFUNG DER HAUT-ALTERUNGSFOLGEN

COSMETIC TREATMENT METHOD FOR FIGHTING AGAINST SKIN AGEING EFFECTS

(84) Etats contractants désignés:
**CH DE ES FR GB IT LI**

(30) Priorité: **29.05.1998 FR 9806822**

(43) Date de publication de la demande:
**14.03.2001 Bulletin 2001/11**

(73) Titulaire: **GUERLAIN Société Anonyme
75008 Paris (FR)**

(72) Inventeurs:
• **BONTE, Frédéric
  F-45100 Orleans (FR)**
• **DUMAS, Marc
  45100 Orleans (FR)**
• **HEUSELE, Catherine
  F-91470 Limours (FR)**
• **LE BLAY, Jacques
  F-28300 Leves (FR)**

(74) Mandataire: **Portal, Gérard
  Cabinet Beau de Loménie
  158, rue de l'Université
  75340 Paris Cedex 07 (FR)**

(56) Documents cités:
WO-A-94/22421     WO-A-97/09963
WO-A-98/19664     FR-A- 2 406 438
FR-A- 2 669 225     FR-A- 2 704 390
FR-A- 2 713 483     FR-A- 2 735 981
US-A- 4 938 969     US-A- 5 804 168

EP 1 082 098 B1

## Description

La présente invention a pour objet un procédé de traitement cosmétique pour lutter contre les effets du vieillissement cutané ainsi que de nouvelles compositions cosmétiques particulièrement adaptées à sa mise en oeuvre.

On sait que la jonction dermo-épidermique (JDE) est une structure complexe qui assure la cohésion et les échanges entre le derme et l'épiderme, indispensables au bon fonctionnement de la peau.

DISCUSSION DE L'ART ANTERIEUR

Il est connu, par le document WO 98/19664, l'utilisation dans une application cosmétique d'un extrait de Potentilla erecta comme agent destiné à améliorer la cohésion entre le derme et l'épiderme ou à stimuler la formation de collagène VII dans le but de prévenir ou de retarder l'apparition des signes de vieillissement cutané. Ce document ne divulgue pas une utilisation d'un sel ou complexe divalent de magnésium ou de zinc, comme agent favorisant l'adhésion des kératinocytes de la couche basale épidermique à la jonction dermo-épidermique.

Le document US-A-4 938 969 concerne une méthode de traitement pour diminuer la profondeur des rides de la peau due au vieillissement intrinsèque ou photo-induit par l'application d'une composition contenant une combinaison critique de 2 à 20 % d'acide ascorbique, de 1 à 10 % de tyrosine et 0,5 à 5 % d'un sel de zinc non toxique.

Ainsi, ce document ne divulgue pas l'utilisation d'un sel de zinc comme agent favorisant l'adhésion des kératinocytes de la couche basale épidermique à la jonction dermo-épidermique, ni la combinaison revendiquée.

Le document WO-94/22421 concerne l'utilisation de sels de magnésium dans des préparations à usage externe pour réduire les dommages de la peau dus aux UV.

Le document FR-2 713 483 concerne une composition cosmétique caractérisée en ce qu'elle renferme un mélange comprenant de la bétaïne ou de la méthionine, de l'ATP, ou un système générateur d'ATP, un sel de magnésium, un sel de potassium, un extrait d'eurotiale. Ce document ne divulgue pas l'utilisation d'un sel de magnésium, comme agent favorisant l'adhésion des kératinocytes de la couche basale épidermique, comme cela est revendiqué dans le cadre de l'invention.

Le document WO 97/09963 décrit une composition pour un traitement dermatologique visant à traiter la peau du patient par l'exfoliation des couches superficielles, cette composition contenant un agent de visualisation, un tensioactif et un acide ou un équivalent d'acide.

Le document US 5 804 168 concerne une composition pharmaceutique pour le traitement des dommages subis par la peau suite à une exposition au soleil comprenant un antioxydant, un anti-inflammatoire et un composant stimulant le système immunitaire.

Le document FR 2 735 981 décrit une composition cosmétique contenant comme agent actif un extrait de plante Foetidia dans le but de stimuler la production de glycosaminoglycannes dans la peau.

Le document FR 2 669 225 concerne l'utilisation de saponines de Medicago dans des compositions cosmétiques.

Les exemples 9 et 10 prévoient un gel coiffant anti-chute ou traitant contre l'alopécie séborrhéique contenant une complexe protéine-zinc, cette combinaison étant réalisée dans un but totalement différent de celui de l'invention.

Il a été découvert, et ceci constitue le fondement de la présente invention qu'il était possible de ralentir ou traiter le vieillissement cutané, en particulier de diminuer la profondeur des rides et/ou ralentir leur apparition et/ou restaurer la tonicité et l'élasticité de la peau et/ou ralentir la diminution de tonicité et d'élasticité de la peau, par un procédé de traitement cosmétique répondant à un nouveau concept consistant à favoriser au moyen d'un agent cosmétiquement acceptable, l'adhésion des kératinocytes de la couche basale épidermique à la jonction dermo-épidermique, notamment au collagène de type IV, encore désigné par collagène IV, constituant majeur de ladite jonction dermo-épidermique.

Ainsi, dans son aspect le plus général, la présente demande vise à couvrir un procédé de traitement cosmétique pour ralentir ou traiter le vieillissement cutané, en particulier pour diminuer la profondeur des rides et/ou pour ralentir leur apparition et/ou pour restaurer la tonicité et l'élasticité de la peau et/ou ralentir la diminution de tonicité et d'élasticité de la peau, caractérisé en ce que l'on applique sur la peau une quantité efficace d'au moins un agent favorisant l'adhésion des kératinocytes de la couche basale épidermique à la jonction dermo-épidermique, notamment au collagène IV de ladite jonction, choisi parmi un chlorure de magnésium ou de zinc ou un sel ou un complexe divalent de magnésium ou de zinc avec un acide aminé ou un alpha-hydroxy acide aliphatique en $C_2$-$C_{12}$, ou un mélange de sel ou de complexe ; ledit agent favorisant l'adhésion précitée étant appliqué sous forme d'une composition le contenant en une quantité comprise entre entre 0,001 et 1 % en poids par rapport au poids total de la composition, soit en association avec une quantité efficace d'au moins un agent stimulant la synthèse de collagène IV, choisi parmi les soyasaponines et les soyasapogénols, de préférence de type A et de type B, et les extraits végétaux riches en de tels composés,; soit en association avec une quantité efficace d'au moins un agent stimulant la synthèse de collagène VII, choisi parmi un extrait de Potentilla erecta, un extrait de Bertholletia, en particulier de Bertholletia excelsa, soit en association avec une combinaison des deux.

Il a, par ailleurs, été montré que des résultats particulièrement remarquables sont obtenus dans le cadre de la présente invention, lorsque l'agent favorisant l'adhésion précité est appliqué en association avec une quantité efficace d'au moins un agent stimulant la synthèse de collagène IV et/ou avec une quantité efficace d'au moins un agent stimulant la synthèse de collagène VII.

Par l'expression "agent stimulant la synthèse de collagène IV ou de collagène VII", on entend dans le cadre de la présente description, tout agent susceptible de produire ou de maintenir une quantité élevée de collagène IV dans la jonction dermo-épidermique, soit par une augmentation de la biosynthèse, soit par une inhibition des enzymes dégradant les protéines constituant ce produit.

Préférentiellement, le sel ou le complexe de métal divalent est ledit acide aminé est choisi parmi l'acide aspartique, l'asparagine, la proline, l'acide glutamique, la méthionine, la leucine, l'histidine ou la lysine, et l'alpha-hydroxy-acide aliphatique en $C_2$-$C_{12}$, est choisi parmi l'acide citrique, l'acide glycolique, l'acide gluconique, l'acide malique, l'acide lactique ou l'acide hydroxy-2-butyrique.

Selon un mode de réalisation actuellement préféré de l'invention, ledit sel ou complexe de métal divalent est l'aspartate de magnésium.

Selon une caractéristique particulière du procédé conforme à la présente invention, l'agent favorisant l'adhésion précité est appliqué sous forme d'une composition le contenant en une quantité comprise entre 0,0001 et 5 %, de préférence entre 0,001 et 1 % en poids, par rapport au poids total de la composition.

Tout agent stimulant la synthèse de collagène IV peut être utilisé dans le cadre du procédé conforme à la présente invention.

Selon un mode de réalisation actuellement préféré, l'agent stimulant la synthèse de collagène IV est choisi parmi les soyasaponines et les soyasapogénols, de préférence de type A et de type B, et les extraits végétaux riches en de tels composés, de préférence les extraits de soja (Glycine max) ou de luzerne (Medicago sativa).

Selon un autre mode de réalisation préféré, l'agent stimulant la synthèse de collagène IV est un totum de saponines de racines de Medicago sativa.

De même, tout agent stimulant la synthèse de collagène VII peut être utilisé dans le cadre de la présente invention.

Selon un mode de réalisation actuellement préféré, l'agent stimulant la synthèse de collagène VII est un extrait de Potentilla erecta.

Selon un autre mode de réalisation préféré, l'agent stimulant la synthèse de collagène VII est un extrait de Bertholletia en particulier de Bertholletia excelsa.

Selon un second aspect, la présente demande vise à couvrir de nouvelles compositions cosmétiques particulièrement adaptées à la mise en oeuvre du procédé décrit précédemment, telles que définies aux revendications de composition.

Ces compositions sont essentiellement caractérisées par le fait qu'elles contiennent une quantité efficace d'au moins un agent favorisant l'adhésion des kératinocytes de la couche basale épidermique à la jonction dermo-épidermique, notamment au collagène IV de ladite jonction, ledit agent étant choisi parmi les sels ou complexes de magnésium ou de zinc en association avec une quantité efficace d'au moins un agent stimulant la synthèse de collagène IV et/ou une quantité efficace d'au moins un agent stimulant la synthèse de collagène VII, ces compositions étant telles que définies dans les revendications.

Dans ces compositions, les différents agents favorisant l'adhésion, stimulant la synthèse de collagène IV ou la synthèse de collagène VII sont tels que décrits précédemment dans le cadre de la description générale du procédé conforme à l'invention.

Les compositions de l'invention pourront comprendre en outre avantageusement au moins une substance favorisant la synthèse des constituants de la matrice extracellulaire de la peau.

Par ailleurs, les compositions selon l'invention peuvent contenir, en outre au moins une substance choisie dans le groupe constitué par les vitamines, en particulier les vitamines du groupe A (rétinol) et C et leurs dérivés tels que les esters notamment les palmitates et propionates, les tocophérols, les xanthines, en particulier la caféine ou la théophylline, les rétinoides, en particulier la vitamine A acide, les extraits de Centella asiatica, les acides asiatiques, madécassiques et leurs dérivés glycosylés tels que l'asiaticoside ou le madécassoside, les extraits de Siegesbeckia orientais, les extraits de Commiphora mukul et les extraits d'Eriobotrya japonica, les dérivés de silicium cosmétiquement acceptables tels que des polysiloxanes, des silanols et des silicones, les alpha-céto-acides aliphatiques en Cs-Ci2, en particulier l'acide pyruvique, les alpha-hydroxy-acides aliphatiques en C2-Ci2, en particulier l'acide citrique, l'acide glycolique, l'acide malique et l'acide lactique, les acides aminés, en particulier l'arginine, la citrulline et la thréonine, les céramides, les glycocéramides, les dérivés de sphingosine, en particulier les céramides de type II et III, les phospholipides, la forskoline et ses dérivés, les extraits de Coleus, les extraits de Tephrosia, les inhibiteurs d'élastase en particulier l'acide ellagique, les peptides de soja, les inhibiteurs de collagénase en particulier les peptides et les extraits végétaux tels que les extraits de racine de Coptidis, les extraits de racine de Scutellaria baicalensis Georgi, les flavonoïdes tels que la wogonine, la baicaline et la baicaléine, les extraits hydro-éthanoliques de feuilles de Ginkgo biloba, de Mosla chinensis, de Salvia officinalis, de Cinnamomum cassia, les extraits cathéchiques de Camellia sinensis et les extraits aqueux de coques de fèves de Theobroma cacao, les anti-inflammatoires en particulier les inhibiteurs de phospholipase A2, les apaisants en particulier les extraits de réglisse, l'acide glycyrrhétinique, le glycyrrhizinate d'ammonium, les agents hydratants en particulier les polyols, le propylène glycol, le butylène glycol, le glycérol, l'acide hyaluronique, les agents anti-vergetures, en particulier les extraits de Marron d'Inde et l'escine, les agents protégeant ou améliorant la microcirculation, en particulier les bioflavonoïdes de Ginkgo biloba, l'isodon, les extraits d'Ami visnaga, la visnadine, la ruscogénine, les antiradicalaires en particulier les polyphénols tels que les OPC (Oligomères Procyanidoliques) et leurs dérivés, des extraits végétaux en particulier des extraits de Curcuma longa, les agents anti-séborrhéiques tels qu'un inhibiteur de 5-alpha-réductase, en particulier un extrait de Pygeum africanum, et les agents stimulant la microcirculation sanguine, tels que la cépharanthine et le nicotinate de méthyle.

Les compositions selon l'invention peuvent avantageusement contenir des substances protégeant la peau des effets nocifs du soleil telles que les filtres solaires seuls ou en combinaison, notamment les filtres UV A et les filtres UV B, en particulier les oxydes de titane et les oxydes de zinc, l'oxybenzone, le Parsol MCX, le Parsol 1789 et les filtres d'origine végétale, les substances limitant les dommages causés à l'ADN, en particulier celles limitant la formation de dimères de thymine tel que l'acide ascorbique et ses dérivés et/ou le Photonyl®, et les substances contribuant à l'élimination des taches de vieillissement tels que les inhibiteurs de la synthèse de mélanine ou de tyrosinase.

D'autres buts, caractéristiques et avantages de l'invention apparaîtront clairement à l'homme de l'art à la lumière de la description explicative qui va suivre faite en référence à plusieurs exemples rapportant des essais effectués, ainsi que des exemples de formulations cosmétiques donnés simplement à titre d'illustration et qui ne sauraient donc en aucune façon limiter la portée de l'invention.

Dans les exemples, tous les pourcentages sont donnés en poids sauf indication contraire.

## EXEMPLE 1

**Essai d'adhérence de kératinocytes humains normaux sur collagène de type IV à l'aide d'un agent selon l'invention favorisant l'adhésion des kératinocytes à la jonction dermo-épidermique, constitué dans cet essai par un chlorure ou un aspartate de magnésium.**

Le présent essai a pour but de démontrer l'efficacité d'un agent favorisant l'adhésion des kératinocytes à la jonction dermo-épidermique selon l'invention, de préférence constitué par un chlorure ou un aspartate de magnésium.

Dans ce cadre, on réalise cet essai de la manière suivante :

1. Tapissage des surfaces d'adhésion avec du collagène de type IV

Des puits de micro-plaques (Falcon) sont recouverts avec 6 $\mu$g/cm$^2$ de collagène humain de type IV, (de chez Sigma) stérile.

Chaque puits est ensuite incubé pendant une nuit à + 4°C avec une solution d'albumine bovine, BSA de chez Sigma à 4 mg/ml.

Les puits sont ensuite rincés deux fois avec un tampon phosphate, PBS (Phosphate Buffered Saline) de chez Gibco.

2. Préparation des cultures de kératinocytes humains normaux

Les cellules épidermiques sont obtenues à partir de peau chirurgicale saine provenant de la zone mammaire d'un donneur caucasien de sexe féminin âgé de 53 ans.

Les fragments de peau sont incubés dans de la trypsine 0,25 % p/v pendant 18 heures à + 4°C permettant la séparation derme/épiderme et l'obtention par agitation, d'une suspension de cellules épidermiques. La neutralisation de la trypsine s'effectue avec du sérum de veau foetal, SVF de chez Gibco.

Les cellules sont ensemencées dans des flacons définissant une surface de 75 cm$^2$ et cultivées dans du milieu de prolifération pour kératinocytes K-SFM de chez Gibco, jusqu'à confluence où elles seront sous-cultivées.

Les cellules utilisées pour les expériences d'adhérence sur le substrat collagénique n'ont pas été sous-cultivées au-delà de la première sous-culture (dénommées P0 ou P1).

3. Traitement des kératinocytes avec le produit de l'invention constitué soit par du chlorure soit par de l'aspartate de magnésium.

De la trypsine (trypsine-EDTA 0,1 % - 0,02 % p/v de chez Gibco) est ajoutée aux cultures de kératinocytes, et la suspension cellulaire est placée dans du milieu E 199 de chez Gibco complémentée de 2 mM de L-glutamine, 4 mg/ml de BSA et contenant 0,25 - 0,5 - 1 mM,, de chlorure de magnésium ou 0,25 mM d'aspartate de magnésium, selon l'invention.

Les kératinocytes sont alors incubés pendant 30 minutes à + 4 °C avant de procéder à l'étape d'adhérence au substrat tapissé de collagène IV.

### 4. Mesure de l'adhérence des kératinocytes au collagène de type IV

Dans chaque puits, les kératinocytes sont ensemencés à la densité de 93000 cellules/cm$^2$ dans du milieu E 199 de chez Gibco contenant 2 mM de L-glutamine de chez Gibco et 4 mg/ml de BSA (Bovine Serum Albumin). Après une heure d'incubation à + 4 °C, les puits sont rincés avec du PBS, les cellules adhérentes sont ensuite lysées par de la soude 0,1 N et les protéines cellulaires sont alors quantifiées par la méthode colorimétrique et l'acide bicinchoninique (BCA de chez Sigma).

Parallèlement, un étalonnage est réalisé à l'aide de la BSA solubilisée dans de la soude 0,1 N permettant de convertir les valeurs de densité optique (DO) en microgrammes ($\mu$g) de protéines par puits.

### 5. Analyse statistique

L'adhérence A est exprimée en microgrammes de protéines cellulaires par puits de culture et les valeurs présentées au tableau I correspondent à une valeur moyenne obtenue à partir de 6 puits par concentration de produits, à savoir d'une part, des cellules non traitées, des cellules traitées avec une concentration de 0,25 mM de chlorure ou d'aspartate de magnésium, des cellules traitées avec une concentration de 0,5 mM de chlorure de magnésium et des cellules traitées avec une concentration de 1 mM de chlorure de magnésium.

Ces valeurs d'adhérence entre cellules traitées et non traitées ont été comparées par le test t de Student au seuil p = 0,05 pour juger de leur niveau de significativité.

Les résultats obtenus par l'expérience sur les kératinocytes d'un donneur de 53 ans sont répertoriés au tableau I ci-après :

### TABLEAU I

|  | A | Ecart type | Test t de Student |
|---|---|---|---|
| Témoin | 3,52 | 0,9 | |
| Chlorure de magnésium (0,25 mM) | 4,32 | 0,7 | Non significatif (p=0,12) |
| Chlorure de magnésium (0,5 mM) | 4,54 | 1,1 | Non significatif (p=0,1) |
| Chlorure de magnésium (1 mM) | 4,57 | 0,75 | Significatif (p=0,05) |
| Aspartate de magnésium (0.25 mM) | 5,39 | 0,8 | Significatif (p=0,004) |
| Où A = Adhérence en $\mu$g de protéine par puits (moyenne) | | | |

A partir des résultats répertoriés au tableau I ci-dessus, on observe par rapport aux cultures témoins une augmentation de l'adhérence des kératinocytes au collagène de type IV en présence de chlorure de magnésium dès la concentration de 0,25 mM mais celle-ci n'est statistiquement significative qu'à partir de 1 mM.

Le taux d'augmentation de l'adhérence obtenu avec le chlorure de magnésium à la concentration de 1 mM est de +31 %.

En ce qui concerne l'aspartate de magnésium, celui-ci favorise également l'adhérence des kératinocytes mais plus fortement que ne le fait le chlorure de magnésium. En effet dès la concentration de 0,25 mM, l'augmentation de l'adhérence est hautement significative.

Le taux d'augmentation de l'adhérence obtenu avec l'aspartate de magnésium à la concentration de 0,25 mM est de +54 %.

Dans ces conditions, on constate ainsi que ces sels de magnésium, et plus particulièrement l'aspartate de magnésium, sont particulièrement intéressants car ils produisent des résultats hautement significatifs à de faibles doses, ce qui permet d'envisager un emploi à des concentrations faibles, présentant une bonne sécurité d'utilisation.

**Exemple 2 de l'invention**

1. Composition de crème anti-rides.

| | |
|---|---|
| L-aspartate de magnésium | 0,3 g |
| Extrait sec de Potentilla erecta | 0,01 g |
| Acide hyaluronique (sel de sodium) | 0,06 g |
| Glycérol | 5,15 g |
| Extrait sec total de Centella asiatica | 0,1 g |
| Solution de palmitate de vitamine A (à 1 Million UI/g) | 0,1 g |
| Acétate de vitamine E | 0,5 g |
| Extrait sec de Perilla | 0,5 g |
| Excipient émulsionné H/E plus parfum et conservateurs | qsp 100g |

2. Essai de cette composition cosmétique pour évaluer son efficacité anti-rides.

A- Principe

Afin d'évaluer l'efficacité anti-rides de ce produit cosmétique au niveau de "la patte d'oie" des répliques négatives de peau sont effectuées au temps 0, puis après 28 jours d'application bi-quotidienne de la composition ci-dessus, sous forme d'une crème.
Ces répliques, éclairées par une lumière rasante générant des ombres portées derrière chaque ride, sont analysées à l'aide d'un logiciel d'analyse d'images dénommé "Quantirides" disponible dans le commerce et mis au point par la société MONADERM (Monaco).

B- Appareillage

B.1- Pour la prise d'empreintes

On utilise des couronnes adhésives de chez 3M, diamètre interne : 24 mm, diamètre externe : 40 mm.
On utilise pour la prise d'empreintes du produit Silflo® de chez FLEXICO UK à base d'un polymère de silicone combiné à un catalyseur.

B.2- Pour l'analyse des empreintes

On utilise une caméra type COHU 4910-RS 170 et CCIR Monochrome CCD caméra haute définition et à très faibles bruits munie d'un objectif à focal fixe et d'un zoom manuel Lens 18 X 108 mm F 2,5 ;

- une carte d'acquisition image haute résolution en temps réel,
- une lampe éclairage rasant Monaspot à 35° d'incidence,
- un statif Kaiser RS 1, un plateau noir mate anti-reflet 450 X 500 mm et colonne de 1.000 mm graduée en centimètres réglable en hauteur munie d'un bras de reproduction RA1,
- un support spécifique pour poser et orienter les répliques,
- le logiciel Quantirides précité,
- un micro-ordinateur et une imprimante.

C- Protocole

1. Sujets volontaires

30 sujets âgés de 34 à 59 ans ont été sélectionnés et répartis de la façon suivante : 29 femmes et 1 homme.

## 2. Produit testé

Le produit testé est la composition sous forme de crème décrite ci-dessus au paragraphe 1.

## 3. Application

La composition cosmétique est appliquée deux fois par jour, matin et soir, sur toute une zone temporale du visage (patte d'oie) pendant 28 jours. La quantité appliquée correspond aux habitudes d'utilisation et peut être estimée à environ 1,5 à 2 mg par cm². L'autre zone temporale "non traitée" sert de témoin.

Durant les trois jours précédant le début du test et pendant toute sa durée, aucun autre produit cosmétique n'est utilisé sur la zone traitée et la zone témoin.

## 4. Conditions expérimentales

Température : de 20 à 22°C
Hygrométrie : de 40 à 50 % HR

Une empreinte des zones témoins est traitée et réalisée au temps 0 et après 28 jours de traitement. On positionne sur la zone d'étude une couronne adhésive. Une fine couche de Silflo® mélangée extemporanément à quelques gouttes de catalyseur (3 gouttes pour 3 g de Silflo®) est appliquée à l'intérieur de la zone délimitée par la rondelle. La pâte doit être soigneusement étalée afin d'éviter le formation de bulles d'air. Après polymérisation de la pâte, 4 minutes 30 de séchage, la rondelle est décollée de la peau, entraînant avec elle la réplique. A la fin de l'étude, ces empreintes sont analysées à l'aide du logiciel Quantirides.

## D. Paramètres étudiés

Pour chaque sujet et pour chaque côté du visage, à J0 (la veille de la première application et J28 (28ème jour d'application), le traitement des empreintes par l'analyseur d'images a permis de calculer les paramètres suivants, représentatifs de l'état de ridulation de la peau :

a) la surface totale des rides en mm²,
b) le nombre de rides,
c) la longueur totale de rides en mm,
d) la longueur moyenne en mm,
e) la profondeur moyenne en $\mu$m,

## E. Exploitation des résultats : évolution du côté traité et du côté témoin

## 1. Calcul

### Variation moyenne des paramètres

On calcule pour chaque site et chaque paramètres :

$$Variation.(\%) = \left( \frac{m(t) - m(0)}{m(0)} \right) \times 100$$

Avec : m (t) = valeur moyenne du paramètre étudié au temps t
m (0) = valeur moyenne du paramètre étudié au temps 0

## 2. Significativité statistique

### Test de Wilcoxon

On utilise le test non paramétrique de Wilcoxon permettant de tenir compte du petit nombre de sujets et applicable à l'étude des paramètres biologiques in-vivo sur l'homme.

On effectue une comparaison des séries appariées de la façon suivante : on forme pour chaque paire la différence puis on classe les différences par valeur absolue croissante en mentionnant par ailleurs pour chacune si elle positive ou négative, les différences nulles sont éliminées.

Les quantités à considérer sont :

M = somme des rangs de différence de signe moins
P = somme des rangs de différence de signe plus
T = le plus petit des deux totaux M ou P

La limite de significativité admise pour n < à 10 personnes est inférieure à 10 %.

La limite de significativité admise pour n $\geq$ à 10 personnes est inférieure à 5 %.

Le test de Wilcoxon a été effectué sur la différence ($m(t) - m(0)$) aux différents temps sur les deux sites pour comparer l'évolution du site traité par rapport au site témoin.

Un test de Wilcoxon a été effectué sur les valeurs brutes au temps 0 ($m(0)$) pour comparer l'évolution de chaque site dans le temps.

3. Résultats

Les résultats obtenus par l'expérience sont répertoriés dans le tableau II ci-après.

Les sites témoins et traités sont comparables au temps 0.

Les valeurs portées en colonne 4 sous le titre "variation totale" correspondent à la différence entre la variation chez les sujets traités et la variation chez les sujets témoins.

TABLEAU II

| | Variation témoin % | Variation traité % | Variation totale % | Statistiques (Wilcoxon) |
|---|---|---|---|---|
| **Surface totale des rides (mm$^2$)** | 6,6 | - 19,7 | -26,3 | Significatif (p = 0,015) |
| **Nombre de rides** | 16,5 | - 14,3 | - 30,8 | Significatif (p = 0,004) |
| **Longueur totale (mm)** | 12,9 | - 16,7 | - 29,6 | Significatif (p = 0,0014) |
| **Longueur moyenne (mm)** | -2,6 | - 1,7 | 0,9 | Non significatif |
| **Profondeur moyenne ($\mu$m)** | 0,3 | - 2,0 | - 2,3 | Non significatif |

E. Conclusion

Après application bi-quotidienne pendant 28 jours de la composition selon l'invention en comparant l'évolution de la patte d'oie traitée par rapport à la patte d'oie témoin, on constate une diminution significative des rides et des ridules et le ralentissement de leur formation : la surface totale des rides diminue de 26 %, leur nombre de 31 % et la longueur totale de 30 %.

**Exemple 3 de l'invention** : crème anti-rides de nuit E/H.

| | | |
|---|---|---|
| Aspartate de magnésium | 0,3 | g |
| Extrait sec de Potentilla erecta | 1 | g |
| Glycérol | 5 | g |
| Propylèneglycol | 2 | g |
| Céramide III | 0,04 | g |
| Filtres UV | 9 | g |
| Méthyl silanol mannuronate | 0,05 | g |
| Extrait sec de Perilla frutescens | 1 | g |

(suite)

| | | |
|---|---|---|
| Extrait sec de Centella asiatica | 0,5 | g |
| Peptide de soja | 1 | g |
| Palmitate de rétinol | 0,2 | g |
| Excipient émulsionné E/H | qsp. 100 | g |

**Exemple 4 de l'invention** : crème anti-relâchement, ralentit et combat l'apparition des rides.

| | | |
|---|---|---|
| Aspartate de magnésium | 0,2 | g |
| Glycérol | 5 | g |
| Propylèneglycol | 2 | g |
| Céramide II | 0,04 | g |
| Parsol MCX | 5 | g |
| Oxybenzone | 3 | g |
| Méthyl silanol mannuronate | 0,05 | g |
| Madécassoside | 0,5 | g |
| Rétinol | 4000 | UI |
| Saponines de Medicago sativa | 0,02 | g |
| Palmitate de rétinol | 0,04 | g |
| Excipient émulsion H/E | qsp. 100 | g |

**Exemple 5 de l'invention** : gel tenseur anti-rides.

| | | |
|---|---|---|
| Gluconate de zinc | 0,3 | g |
| Extrait sec de Bertholletia excelsa | 0,3 | g |
| Soyasaponine de soja | 0,05 | g |
| Palmitate de rétinol | 0,06 | g |
| Acétate d'alpha-tocophérol | 0,1 | g |
| Acide lactique | 1,5 | g |
| Acide glycolique | 0,2 | g |
| Ethanol | 5 | g |
| Excipient gel | qsp 100 | g |

**Revendications**

1. Procédé de traitement cosmétique pour ralentir ou traiter le vieillissement cutané, en particulier pour diminuer la profondeur des rides et/ou pour ralentir leur apparition et/ou pour restaurer la tonicité et l'élasticité de la peau et/ou ralentir la diminution de tonicité et d'élasticité de la peau, **caractérisé en ce que** l'on applique sur la peau une quantité efficace d'au moins un agent favorisant l'adhésion des kératinocytes de la couche basale épidermique à la jonction dermo-épidermique, notamment au collagène IV de ladite jonction, choisi parmi un chlorure de magnesium ou de zinc ou un sel ou un complexe divalent de magnésium ou de zinc avec un acide aminé ou un alpha-hydroxy-acide aliphatique en $C_2$-$C_{12}$, ou un mélange de sel ou de complexe; ledit agent favorisant l'adhésion précitée étant appliqué sous forme d'une composition le contenant en une quantité comprise entre 0,001 et 1 % en poids par rapport au poids total de la composition, soit en association avec une quantité efficace d'au moins un agent stimulant la synthèse de collagène IV, choisi parmi les soyasaponines et les soyasapogénols, de préférence de type A et de type B, et les extraits végétaux riches en de tels composés,; soit en association avec une quantité efficace d'au moins un agent stimulant la synthèse de collagène VII, choisi parmi un extrait de Potentilla erecta, un extrait de Bertholletia, soit en association avec une combinaison des deux.

**2.** Procédé selon la revendication 1, **caractérisé en ce que** l'acide aminé est choisi parmi l'acide aspartique, l'asparagine, la proline, l'acide glutamique, la méthionine, la leucine, l'histidine et la lysine, et l'alpha-hydroxy-acide aliphatique en $C_2$-$C_{12}$ est choisi parmi l'acide citrique, l'acide glycolique, l'acide gluconique, l'acide malique, l'acide lactique et l'acide hydroxy-2-butyrique.

**3.** Procédé selon la revendication 1 ou 2 **caractérisé en ce que** ledit sel ou complexe de métal divalent est l'aspartate de magnésium ou le chlorure de magnésium.

**4.** Procédé selon l'une des revendications 1 à 3, **caractérisé en ce que** l'agent stimulant la synthèse de collagène IV est un extrait de soja (Glycine max) ou de luzerne (Medicago sativa), ou un totum de saponines de racines de Medicago sativa.

**5.** Procédé selon l'une des revendications 1 à 4, **caractérisé en ce que** l'agent stimulant la synthèse de collagène VII est un extrait de Bertholletia excelsa.

**6.** Composition cosmétique pour ralentir ou traiter le vieillissement cutané, en particulier pour diminuer la profondeur des rides et/ou pour ralentir leur apparition et/ou pour restaurer la tonicité et l'élasticité de la peau et/ou ralentir la diminution de tonicité et d'élasticité de la peau, **caractérisée en ce qu'**elle contient une quantité efficace comprise entre 0,001 et 1 % en poids par rapport au poids total de la composition d'au moins un agent favorisant l'adhésion des kératinocytes de la couche basale épidermique à la jonction dermo-épidermique, notamment au collagène IV de ladite jonction, choisi parmi un chlorure de magnésium ou de zinc ou un sel ou un complexe de magnésium ou de zinc avec un acide organique cosmétiquement acceptable choisi parmi un acide aminé, et un alpha-hydroxy-acide aliphatique en $C_2$-$C_{12}$ en association avec une quantité efficace d'au moins un agent stimulant la synthèse de collagène IV choisi parmi les soyasaponines et les soyasapogénols, de préférence de type A et de type B, et les extraits végétaux riches en de tels composés,

**7.** Composition cosmétique pour ralentir ou traiter le vieillissement cutané, en particulier pour diminuer la profondeur des rides et/ou pour ralentir leur apparition et/ou pour restaurer la tonicité et l'élasticité de la peau et/ou ralentir la diminution de tonicité et d'élasticité de la peau, **caractérisée en ce qu'**elle contient une quantité efficace comprise entre 0,001 et 1 % en poids par rapport au poids total de la composition d'au moins un agent favorisant l'adhésion des kératinocytes de la couche basale épidermique à la jonction dermo-épidermique, notamment au collagène IV de ladite jonction, choisi parmi un chlorure de magnésium ou de zinc ou un sel ou un complexe de magnésium ou de zinc avec un acide organique cosmétiquement acceptable choisi parmi un acide aminé, et un alpha-hydroxy-acide aliphatique en $C_2$-$C_{12}$ en association avec une quantité efficace d'au moins un agent stimulant la synthèse de collagène VII choisi parmi un extrait de Potentilla erecta, un extrait de Bertholletia, ou une combinaison des deux.

**8.** Composition cosmétique pour ralentir ou traiter le vieillissement cutané, en particulier pour diminuer la profondeur des rides et/ou pour ralentir leur apparition et/ou pour restaurer la tonicité et l'élasticité de la peau et/ou ralentir la diminution de tonicité et d'élasticité de la peau, **caractérisée en ce qu'**elle contient une quantité efficace comprise entre 0,001 et 1 % en poids par rapport au poids total de la composition d'au moins un agent favorisant l'adhésion des kératinocytes de la couche basale épidermique à la jonction dermo-épidermique, notamment au collagène IV de ladite jonction d'au moins un agent favorisant l'adhésion des kératinocytes de la couche basale épidermique à la jonction dermo-épidermique, notamment au collagène IV de ladite jonction, choisi parmi un chlorure de magnésium ou de zinc ou un sel ou un complexe de magnésium ou de zinc avec un acide organique cosmétiquement acceptable choisi parmi un acide aminé, et un alpha-hydroxy-acide aliphatique en $C_2$-$C_{12}$ en association avec une quantité efficace d'au moins un agent stimulant la synthèse de collagène IV choisi parmi les soyasaponines et les soyasapogénols, de préférence de type A et de type B, et les extraits végétaux riches en de tels composés, et une quantité efficace d'au moins un agent stimulant la synthèse de collagène VII choisi parmi un extrait de Potentilla erecta, un extrait de Bertholletia, ou une combinaison des deux.

**9.** Composition cosmétique selon l'une des revendication 6 à 8, **caractérisée en ce que** l'acide aminé précité est choisi parmi l'acide aspartique, l'asparagine, la proline, l'acide glutamique, la méthionine, la leucine ; l'alpha-hydroxy-acide aliphatique en $C_2$-$C_{12}$ est choisi parmi l'acide citrique, l'acide glycolique, l'acide gluconique, l'acide malique, l'acide lactique ou l'acide hydroxy-2-butyrique.

**10.** Composition cosmétique selon l'une des revendications 6 à 9, **caractérisée en ce que** ledit sel ou complexe est l'aspartate de magnésium ou le chlorure de magnésium.

**11.** Composition cosmétique selon l'une des revendications 6 ou 8 à 11, **caractérisée** en ce l'agent stimulant la synthèse de collagène IV est choisi parmi les soyasaponines et les soyasapogénols, de type A et de type B, les extraits végétaux riches en de tels composés, sont choisis parmi les extraits de soja (Glycine max) ou de luzerne (Medicago sativa), et un totum de saponines de racines de Medicago sativa.

**12.** Composition cosmétique selon l'une des revendications 7, 8 à 11, **caractérisée en ce que** l'agent stimulant la synthèse de collagène VII est un extrait de Bertholletia excelsa.

**13.** Composition cosmétique selon l'une des revendications 6 à 13, **caractérisée en ce qu'**elle contient en outre au moins une substance choisie dans le groupe constitué par les vitamines, en particulier les vitamines du groupe A (rétinol) et C et leurs dérivés tels que les esters notamment les palmitates et propionates, les tocophérols, les xanthines, en particulier la caféine ou la théophylline, les rétinoides, en particulier la vitamine A acide, les extraits de Centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica, les dérivés de silicium cosmétiquement acceptables tels que des polysiloxanes, des silanols et des silicones, les alpha-céto-acides aliphatiques en $C_3$-$C_{12}$, en particulier l'acide pyruvique, les alpha-hydroxy-acides aliphatiques en $C_2$-$C_{12}$, en particulier l'acide citrique, l'acide glycolique, l'acide malique et l'acide lactique, les acides aminés, en particulier l'arginine, la citrulline et la thréonine, les céramides, les glycocéramides, les dérivés de sphingosine, en particulier les céramides de type II et III, les phospholipides, la forskoline et ses dérivés, les extraits de Coleus, les extraits de Tephrosia, les inhibiteurs d'élastase en particulier l'acide ellagique, les peptides de soja, les inhibiteurs de collagénase en particulier les peptides et les extraits végétaux tels que les extraits de racine de Coptidis, les extraits de racine de Scutellaria baicalensis Georgi, les flavonoïdes tels que la wogonine, la baicaline et la baicaléine, les extraits hydro-éthanoliques de feuilles de Ginkgo biloba, de Mosla chinensis, de Salvia officinalis, de Cinnamomum cassia, les extraits cathéchiques de Camellia sinensis et les extraits aqueux de coques de fèves de Theobroma cacao, les anti-inflammatoires en particulier les inhibiteurs de phospholipase A2, les apaisants en particulier les extraits de réglisse, l'acide glycyrrhétinique, le glycyrrhizinate d'ammonium, les agents hydratants en particulier les polyols, le propylène glycol, le butylène glycol, le glycérol, l'acide hyaluronique, les agents anti-vergetures, en particulier les extraits de Marron d'Inde et l'escine, les agents protégeant ou améliorant la microcirculation, en particulier les bioflavonoïdes de Ginkgo biloba, l'isodon, les extraits d'Ami visnaga, la visnadine, la ruscogénine, les antiradicalaires en particulier les polyphénols tels que les OPC (Oligomères Procyanidoliques) et leurs dérivés, des extraits végétaux en particulier des extraits de Curcuma longa, les agents anti-séborrhéiques tels qu'un inhibiteur de 5-alpha-réductase, en particulier un extrait de Pygeum africanum, et les agents stimulant la microcirculation sanguine, tels que la cépharanthine et le nicotinate de méthyle.

**14.** Composition cosmétique selon l'une des revendications 7 à 15, **caractérisée en ce qu'**elle contient en outre au moins une substance choisie parmi les substances protégeant la peau des effets nocifs du soleil telles que les filtres solaires seuls ou en combinaison, notamment les filtres UV A et les filtres UV B, en particulier les oxydes de titane et les oxydes de zinc, l'oxybenzone et les filtres d'origine végétale, les substances limitant les dommages causés à l'ADN, en particulier celles limitant la formation de dimères de thymine tel que l'acide ascorbique et ses dérivés, et les substances contribuant à l'élimination des taches de vieillissement tels que les inhibiteurs de la synthèse de mélanine ou de tyrosinase.

**Patentansprüche**

**1.** Verfahren zur kosmetischen Behandlung, um die Hautalterung zu verlangsamen oder zu behandeln, insbesondere um die Faltentiefe zu verringern und/oder um ihr Erscheinen zu verlangsamen und/oder um die Spannkraft und die Elastizität der Haut wiederherzustellen und/oder die Verringerung der Spannkraft und der Elastizität der Haut zu verlangsamen, **dadurch gekennzeichnet, dass** eine wirksame Menge von mindestens einem Wirkstoff, der die Adhäsion der Keratinozyten der epidermalen Basalschicht an der dermal-epidermalen Verbindung, insbesondere an dem Kollagen IV der Verbindung, begünstigt, auf die Haut aufgetragen wird, der ausgewählt wird aus einem Magnesium- oder Zinkchlorid oder einem Salz oder einem zweiwertigen Komplex von Magnesium oder Zink mit einer Aminosäure oder einer aliphatischen $C_2$-$C_{12}$-Alpha-Hydroxy-Säure oder einer Mischung aus Salz oder dem Komplex, wobei der vorgenannte Adhäsionsverstärker in Form einer Zusammensetzung, die ihn enthält, in einer Menge zwischen 0,001 und 1 Gew.-%, bezogen auf das Gesamtgewicht der Zusammensetzung, entweder in Verbindung mit einer wirksamen Menge von mindestens einem Wirkstoff, der die Synthese von Kollagen IV anregt, der ausgewählt ist aus den Sojasaponinen und den Sojasapogenolen, vorzugsweise vom Typ A und vom Typ B, und den Pflanzenextrakten, die reich an solchen Verbindungen sind, oder in Verbindung mit einer wirksamen Menge von mindestens einem Wirkstoff, der die Synthese von Kollagen VII anregt, ausgewählt aus einem Extrakt von Potentilla erecta, einem Bertholletia-Extrakt oder in Verbindung mit einer Kombination von beiden, aufgetragen wird.

2. Verfahren gemäß Anspruch 1, **dadurch gekennzeichnet, dass** die Aminosäure ausgewählt wird aus Asparaginsäure, Asparagin, Prolin, Glutaminsäure, Methionin, Leucin, Histidin und Lysin, und die aliphatische $C_2$-$C_{12}$-Alpha-Hydroxy-Säure ausgewählt wird aus Zitronensäure, Glykolsäure, Gluconsäure, Äpfelsäure, Milchsäure und 2-Hydroxybuttersäure.

3. Verfahren gemäß Anspruch 1 oder 2, **dadurch gekennzeichnet, dass** das Salz oder der Komplex von zweiwertigem Metall Magnesiumaspartat oder Magnesiumchlorid ist.

4. Verfahren gemäß einem der Ansprüche 1 bis 3, **dadurch gekennzeichnet, dass** der Wirkstoff, der die Synthese von Kollagen VII anregt, ein Sojaextrakt (Glycine max) oder Luzerne-Extrakt (Medicago sativa) oder ein Totum von Saponinen aus Wurzeln von Medicago sativa ist.

5. Verfahren gemäß einem der Ansprüche 1 bis 4, **dadurch gekennzeichnet, dass** der Wirkstoff, der die Synthese von Kollagen VII anregt, ein Extrakt von Bertholletia excelsa ist.

6. Kosmetische Zusammensetzung, um die Hautalterung zu verlangsamen oder zu behandeln, insbesondere um die Faltentiefe zu verringern und/oder um ihr Erscheinen zu verlangsamen und/oder um die Spannkraft und die Elastizität der Haut wiederherzustellen und/oder die Verringerung der Spannkraft und der Elastizität der Haut zu verlangsamen, **dadurch gekennzeichnet, dass** sie eine wirksame Menge zwischen 0,001 und 1 Gew.-%, bezogen auf das Gesamtgewicht der Zusammensetzung, von mindestens einem Wirkstoff, der die Adhäsion der Keratinozyten der epidermalen Basalschicht an der dermal-epidermalen Verbindung, insbesondere an dem Kollagen IV der Verbindung, begünstigt, der ausgewählt ist aus einem Magnesium- oder Zinkchlorid oder einem Salz oder einem Komplex von Magnesium oder Zink mit einer kosmetisch akzeptablen organischen Säure, ausgewählt aus einer Aminosäure und einer aliphatischen $C_2$-$C_{12}$-Alpha-Hydroxy-Säure, in Verbindung mit einer wirksamen Menge von mindestens einem Wirkstoff, der die Synthese von Kollagen IV anregt, der ausgewählt ist aus den Sojasaponinen und den Sojasapogenolen, vorzugsweise vom Typ A und vom Typ B, und den Pflanzenextrakten, die reich an solchen Verbindungen sind, umfasst.

7. Kosmetische Zusammensetzung, um die Hautalterung zu verlangsamen oder zu behandeln, insbesondere um die Faltentiefe zu verringern und/oder um ihr Erscheinen zu verlangsamen und/oder um die Spannkraft und die Elastizität der Haut wiederherzustellen und/oder die Verringerung der Spannkraft und der Elastizität der Haut zu verlangsamen, **dadurch gekennzeichnet, dass** sie eine wirksame Menge zwischen 0,001 und 1 Gew.-%, bezogen auf das Gesamtgewicht der Zusammensetzung, von mindestens einem Wirkstoff, der die Adhäsion der Keratinozyten der epidermalen Basalschicht an der dermal-epidermalen Verbindung, insbesondere an dem Kollagen IV der Verbindung, begünstigt, der ausgewählt ist aus einem Magnesium- oder Zinkchlorid oder einem Salz oder einem Komplex von Magnesium oder Zink mit einer kosmetisch akzeptablen organischen Säure, ausgewählt aus einer Aminosäure und einer aliphatischen $C_2$-$C_{12}$-Alpha-Hydroxy-Säure, in Verbindung mit einer wirksamen Menge von mindestens einem Wirkstoff, der die Synthese von Kollagen VII anregt, der ausgewählt ist aus einem Extrakt von Potentilla erecta, einem Bertholletia-Extrakt oder einer Verbindung von diesen zwei, umfasst.

8. Kosmetische Zusammensetzung, um die Hautalterung zu verlangsamen oder zu behandeln, insbesondere um die Faltentiefe zu verringern und/oder um ihr Erscheinen zu verlangsamen und/oder um die Spannkraft und die Elastizität der Haut wiederherzustellen und/oder die Verringerung der Spannkraft und der Elastizität der Haut zu verlangsamen, **dadurch gekennzeichnet, dass** sie eine wirksame Menge zwischen 0,001 und 1 Gew.-%, bezogen auf das Gesamtgewicht der Zusammensetzung, von mindestens einem Wirkstoff, der die Adhäsion der Keratinozyten der epidermalen Basalschicht an der dermal-epidermalen Verbindung, insbesondere an dem Kollagen IV der Verbindung, begünstigt, der ausgewählt ist aus einem Magnesium- oder Zinkchlorid oder einem Salz oder einem Komplex von Magnesium oder Zink mit einer kosmetisch akzeptablen organischen Säure, ausgewählt aus einer Aminosäure und einer aliphatischen $C_2$-$C_{12}$-Alpha-Hydroxy-Säure, in Verbindung mit einer wirksamen Menge von mindestens einem Wirkstoff, der die Synthese von Kollagen IV anregt, der ausgewählt ist aus den Sojasaponinen und den Sojasapogenolen, vorzugsweise vom Typ A und vom Typ B, und den Pflanzenextrakten, die reich an solchen Verbindungen sind, und einer wirksamen Menge von mindestens einem Wirkstoff, der die Synthese von Kollagen VII anregt, der ausgewählt ist aus einem Extrakt von Potentilla erecta, einem Bertholletia-Extrakt oder einer Verbindung von diesen zwei, umfasst.

**9.** Kosmetische Zusammensetzung gemäß einem der Ansprüche 6 bis 8, **dadurch gekennzeichnet, dass** die oben genannte Aminosäure ausgewählt ist aus Asparaginsäure, Asparagin, Prolin, Glutaminsäure, Methionin, Leucin, die aliphatische $C_2$-$C_{12}$-Alpha-Hydroxy-Säure ausgewählt ist aus Zitronensäure, Glykolsäure, Gluconsäure, Äpfelsäure, Milchsäure und 2-Hydroxybuttersäure.

**10.** Kosmetische Zusammensetzung gemäß einem der Ansprüche 6 bis 9, **dadurch gekennzeichnet, dass** das Salz oder der Komplex Magnesiumaspartat oder Magnesiumchlorid ist.

**11.** Kosmetische Zusammensetzung gemäß einem der Ansprüche 6 oder 8 bis 11, **dadurch gekennzeichnet, dass** der Wirkstoff, der die Synthese von Kollagen VI anregt, ausgewählt ist aus den Sojasaponinen und den Sojasapogenolen, vorzugsweise vom Typ A und vom Typ B, die Pflanzenextrakte, die reich an solchen Verbindungen sind, ausgewählt sind aus dem Sojaextrakt (Glycine max) oder Luzerne-Extrakt (Medicago sativa) und einem Totum von Saponinen aus Wurzeln von Medicago sativa.

**12.** Kosmetische Zusammensetzung gemäß einem der Ansprüche 7 , 8 bis 11, **dadurch gekennzeichnet, dass** der Wirkstoff, der die Synthese von Kollagen VII anregt, ein Extrakt von Bertholletia excelsa ist.

**13.** Kosmetische Zusammensetzung gemäß einem der Ansprüche 6 bis 12, **dadurch gekennzeichnet, dass** sie ferner mindestens eine Substanz enthält, ausgewählt aus der Gruppe, bestehend aus den Vitaminen, insbesondere den Vitaminen der A-Gruppe (Retinol) und der C-Gruppe und deren Derivate wie Ester, insbesondere Palmitate und Propionate, Tocopherole, Xanthine, insbesondere Koffein oder Theophyllin, den Retinoiden, insbesondere Vitamin-A-Säure, den Extrakten von Centella asiatica, den Asiat- und Madecasssäuren und deren glykosylierten Derivaten, wie Asiaticosid oder Madecassosid, den Extrakten von Siegesbeckia orientalis, den Extrakten von Commiphora mukul und den Extrakten von Eriobotyra japonica, den kosmetisch verträglichen Siliciumderivaten wie Polysiloxanen, Silanolen und Siliconen, den aliphatischen $C_3$-$C_{12}$-alpha-Ketosäuren, insbesondere Brenztraubensäure, den aliphatischen $C_2$-$C_{12}$-alpha-Hydroxysäuren, insbesondere Zitronensäure, Glykolsäure, Äpfelsäure und Milchsäure, den Aminosäuren, insbesondere Arginin, Citrullin und Threonin, den Ceramiden, den Glycoceramiden, den Sphingosin-Derivaten, insbesondere den Ceramiden vom Typ II und III, den Phospholipiden, dem Forskolin und dessen Derivaten, den Extrakten von Coleus, den Extrakten von Tephrosia, den Elastaseinhibitoren, insbesondere Ellagsäure, den Sojapeptiden, den Kollagenase-Hemmern, insbesondere den Peptiden und den Pflanzenextrakten, wie beispielsweise die Coptidis Wurzel-Extrakte, den Scutellaria baicalensis Georgi Wurzel-Extrakten, den Flavonoiden wie Wogonin, Baicalin und Baicalein, den hydroethanolischen Extrakten aus den Blättern des Ginkgo Biloba, Mosla chinensis, Salvia officinalis, Cinnamomum cassia, den Cathechin-Extrakten von Camellia sinensis und den wässrigen Extrakten von Theobroma cacao-Kakaobohnenschalten, den Entzündungshemmern, insbesondere den Phospholipase A2-Inhibitoren, den Beruhigungsmitteln, insbesondere den Extrakten von Süßholz, Glycyrrhetinsäure, Ammoniumglycyrrhizinat, den Hydratisierungsmitteln, insbesondere den Polyolen, Propylenglykol, Butylenglykol, Glycerin, Hyaluronsäure, den Mitteln gegen Dehnungsstreifen, insbesondere den Extrakten von Rosskastanie und Aescin, den Mitteln zum Schutz oder zur Verbesserung der Mikrozirkulation, insbesondere den Bioflavonoiden von Ginkgo biloba, Isodon, den Extrakten von Ami visnaga, Visnadin, Ruscogenin, den freien Radikalen, insbesondere den Polyphenolen wie OPC (oligomer Proanthocyanidine) und deren Derivate, Pflanzenextrakten, insbesondere Extrakten von Curcuma longa, den antisborrhoischen Wirkstoffen, wie einem 5-alpha-Reduktase-Inhibitor, insbesondere einem Extrakt von Pygeum africanum, und den Mitteln, die die Mikrozirkulation des Blutes stimulieren, wie Cepharanthin und Methylnikotinat.

**14.** Kosmetische Zusammensetzung gemäß einem der Ansprüche 7 bis 13, **dadurch gekennzeichnet, dass** sie ferner mindestens eine Substanz enthält, die ausgewählt ist aus den Substanzen, die die Haut vor den schädlichen Einwirkungen der Sonne schützen, wie Sonnenschutzmittel einzeln oder in Kombination, insbesondere die UVA-Filter und die UVB-Filter, insbesondere Titanoxide und Zinkoxide, Oxybenzon und die Filter pflanzlichen Ursprungs, den Substanzen, die DNA-Schäden begrenzen, insbesondere jene, die die Bildung von Thymin-Dimeren begrenzen, wie Ascorbinsäure und ihre Derivate, und den Substanzen, die zur Elimination von Altersflecken beitragen, wie die Inhibitoren der Melaninsynthese oder die Tyrosinase-Inhibitoren.

## Claims

**1.** Method of cosmetic treatment for slowing down or treating skin ageing, and in particular for reducing the depth of wrinkles, and/or slowing down their appearance, and/or restoring the tonicity and elasticity of the skin, and/or slowing down the decrease in tonicity and elasticity of the skin, **characterized in that** it is applied to the skin an efficient amount of at least one agent for promoting the adhesion of the keratinocytes of the epidermal basal layer to the dermo-epidermal junction, especially to the collagen IV of said junction, selected from magnesium or zinc chloride, or a divalent magnesium or zinc complex with an amino acid, or an $C_2$-$C_{12}$ aliphatic alpha-hydroxy acid, or a mixture of said salt or of said complex; said agent for promoting the adhesion being applied in a composition containing it in an amount ranging between 0.001 and 1% by weight with respect to the total weight of the composition, either in association with an efficient amount of at least an agent stimulating the synthesis of collagen IV, selected from soya saponins and soya sapogenols, preferably of type A and type B, and plant extracts rich in such compounds, or in association with an efficient amount of an agent stimulating the synthesis of collagen VII, selected from an extract of Potentilla erecta, an extract of Bertholletia, or in association with a combination of both.

2.  Method according to claim 1, **characterized in that** the amino acid is selected from aspartic acid, asparagine, proline, glutamic acid, methionine, leucine, histidine or lysine, or a $C_2$-$C_{12}$ aliphatic alpha-hydroxy acid, particularly citric acid, glycolic acid, gluconic acid, malic acid, lactic acid or 2-hydroxybutyric acid.

3.  Method according to claim 1 or 2, **characterized in that** said divalent metal salt or complex is magnesium aspartate or magnesium chloride.

4.  Method according to one of claims 1 to 3, **characterized in that** the agent stimulating the synthesis of collagen IV is an extract of soya (Glycine max) or of alfalfa (Medicago sativa) or a totum of saponins from roots of Medicago sativa.

5.  Method according to one of claims 1 to 4, **characterized in that** the agent stimulating synthesis of collagen VII is an extract of Bertholletia excelsa.

6.  Cosmetic composition for slowing down or treating skin ageing, and in particular for reducing the depth of wrinkles, and/or slowing down their appearance, and/or restoring the tonicity and elasticity of the skin, and/or slowing down the decrease in tonicity and elasticity of the skin, **characterized in that** it contains an effective amount ranging between 0.001 and 1 weight % with respect to the total weight of the composition, of at least one agent for promoting the adhesion of the keratinocytes of the epidermal basal layer to the dermo-epidermal junction, especially to the collagen IV of said junction, selected from a magnesium or zinc salt or a magnesium or zinc complex with an amino acid, or an $C_2$-$C_{12}$ aliphatic alpha-hydroxy acid, in association with an effective amount of at least one agent stimulating synthesis of collagen IV selected from soya saponins and soya sapogenols, preferably of type A and type B, and plant extracts rich in such compounds.

7.  Cosmetic composition for slowing down or treating skin ageing, and in particular for reducing the depth of wrinkles, and/or slowing down their appearance, and/or restoring the tonicity and elasticity of the skin, and/or slowing down the decrease in tonicity and elasticity of the skin, **characterized in that** it contains an effective amount ranging between 0.001 and 1 weight % with respect to the total weight of the composition, of at least one agent for promoting the adhesion of the keratinocytes of the epidermal basal layer to the dermo-epidermal junction, especially to the collagen IV of said junction, selected from magnesium or zinc salt or magnesium or zinc complex with a cosmetically acceptable organic acid selected from an amino acid, and an $C_2$-$C_{12}$ aliphatic alpha-hydroxy acid, in association with an effective amount of at least one agent stimulating synthesis of collagen VII selected from an extract of Potentilla erecta, an extract of Bertholletia, or in association with a combination of both.

8.  Cosmetic composition for slowing down or treating skin ageing, and in particular for reducing the depth of wrinkles, and/or slowing down their appearance, and/or restoring the tonicity and elasticity of the skin, and/or slowing down the decrease in tonicity and elasticity of the skin, **characterized in that** it contains an effective amount ranging between 0.001 and 1 weight % with respect to the total weight of the composition, of at least one agent for promoting the adhesion of the keratinocytes of the epidermal basal layer to the dermo-epidermal junction, especially to the collagen IV of said junction, selected from a magnesium or zinc salt or a magnesium or zinc complex with a cosmetically acceptable organic acid selected from an amino acid, and an $C_2$-$C_{12}$ aliphatic alpha-hydroxy acid, in association with an effective amount of at least one agent stimulating synthesis of collagen IV selected from soya saponins and soya sapogenols, preferably of type A and type B, and plant extracts rich in such compounds., and an effective amount of at least one agent stimulating synthesis of collagen VII selected from an extract of Potentilla erecta, an extract of Bertholletia, or a combination of both .

9.  Cosmetic composition according to one of claims 6 to 8, **characterized in that** the amino acidis selected from aspartic acid, asparagine, proline, glutamic acid, methionine, leucine, histidine or lysine, or a $C_2$-$C_{12}$ aliphatic alpha-hydroxy acid, particularly citric acid, glycolic acid, gluconic acid, malic acid, lactic acid or 2-hydroxybutyric acid.

**10.** Cosmetic composition according to one of claims 6 to 9, **characterized in that** said salt or complex is magnesium aspartate or magnesium chloride.

**11.** Cosmetic composition according to one of claims 6 or 8 to 11, **characterized in that** the agent stimulating synthesis of collagen IV is selected from soya saponins and soya sapogenols, preferably of type A and type B, plant extracts rich in such compounds, are selected from extracts of soya (Glycine max) or alfalfa (Medicago sativa), and a totum of saponins from roots of Medicago sativa.

**12.** Cosmetic composition according to one of claims 7, 8 to 12, **characterized in that** the agent stimulating synthesis of collagen VII is an extract of Bertholletia excelsa.

**13.** Cosmetic composition according to one of claims 6 to 12, **characterized in that** it also contains at least one substance selected from the group consisting of vitamins, particularly the vitamins of group A (retinol) and group C and derivatives thereof such as the esters, especially the palmitates and propionates, tocopherols, xanthines, particularly caffeine or theophylline, retinoids, particularly vitamin A acid, extracts of Centella asiatica, asiatic and madecassic acids and glycosylated derivatives thereof such as asiaticoside or madecassoside, extracts of Siegesbeckia orientalis, extracts of Commiphora mukul and extracts of Eriobotrya japonica, cosmetically acceptable silicon derivatives such as polysiloxanes, silanols and silicones, $C_3$-$C_{12}$ aliphatic alpha-keto acids, particularly pyruvic acid, $C_2$-$C_{12}$ aliphatic alpha-hydroxy acids, particularly citric acid, glycolic acid, malic acid and lactic acid, amino acids, particularly arginine, citrulline and threonine, ceramides, glycoceramides, sphingosine derivatives, particularly type II and III ceramides, phospholipids, forskolin and derivatives thereof, extracts of Coleus, extracts of Tephrosia, elastase inhibitors, particularly ellagic acid and soya peptides, collagenase inhibitors, particularly plant peptides and extracts such as extracts of roots of Coptidis and extracts of roots of Scutellaria baicalensis Georgi, flavonoids such as wogonin, baicalin and baicalein, aqueous-ethanolic extracts of leaves of Ginkgo biloba, Mosla chinensis, Salvia officinalis and Cinnamomum cassia, catechuic extracts of Camellia sinensis and aqueous extracts of bean shells of Theobroma cacao, anti-inflammatories, particularly phospholipase A2 inhibitors, soothing agents, particularly extracts of liquorice, glycyrrhetinic acid and ammonium glycyrrhizinate, hydrating agents, particularly polyols, propylene glycol, butylene glycol, glycerol and hyaluronic acid, agents for combating stretch marks, particularly extracts of horse chestnut and escin, agents for protecting or improving the microcirculation, particularly bioflavonoids from Ginkgo biloba, isodon, extracts of Ami visnaga, visnadine and ruscogenin, free radical inhibitors, particularly polyphenols such as PCO (procyanidolic oligomers) and derivatives thereof and plant extracts, particularly extracts of Curcuma longa, antiseborrhea agents, such as a 5-alpha-reductase inhibitor, particularly an extract of Pygeum africanum, and stimulants of the microcirculation of the blood, such as cepharanthine and methyl nicotinate.

**14.** Cosmetic composition according to one of claims 7 to 13, **characterized in that** it also contains at least one substance selected from substances for protecting the skin from the harmful effects of the sun, such as solar filters, individually or in combination, especially UV A filters and UV B filters, particularly titanium oxides and zinc oxides, oxybenzone, Parsol MCX, Parsol 1789 and filters of vegetable origin, substances for limiting the damage caused to the DNA, particularly those for limiting the formation of thymine dimers, such as ascorbic acid and derivatives thereof and/or Photonyl®, and substances for contributing to the elimination of liver spots, such as inhibitors of melanin or tyrosinase synthesis.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9819664 A
- US 4938969 A
- WO 9422421 A
- FR 2713483
- WO 9709963 A
- US 5804168 A
- FR 2735981
- FR 2669225